# EUROPEAN PATENT SPECIFICATION

(11) **EP 0 356 573 B1**
(45) Date of publication and mention of the grant of the patent: **07.12.1994**
(21) Application number: 88114391.1
(22) Date of filing: 02.09.1988
(51) Int. Cl.: A61N 1/08, A61N 1/05, A61B 5/04

(54) **Stimulating heart inspection apparatus**
Reizherzuntersuchungsgerät
Dispositif de surveillance de stimulation du coeur

(43) Date of publication of application: 07.03.1990
(73) Proprietor: Nihon Kohden Corporation, Shinjuku-ku Tokyo (JP)
(72) Inventor: Itoh, Meiichi, Sendai-shi Miyagi-ken (JP); Kuriyagawa, Kazuya, Miyagi-gun Miyagi-ken (JP); Adachi, Hitoshi, Nerima-ku Tokyo (JP); Ohno, Kohei, Higashiyamato-shi Tokyo (JP)
(74) Representative: Sajda, Wolf E., Dipl.-Phys.

(56) References cited:
- US-A- 4 596 255
- US-A- 4 708 142

## Description

### BACKGROUND OF THE INVENTION

The invention relates to a heart stimulation inspection apparatus including stimulation means in which electrical stimulation waves, generated by electrical stimulation wave generating means, for effecting pacing in accordance with an externally set stimulation pattern, are input through a catheter and the intracardiac electrocardiogram signals induced in the catheter are amplified and recorded on a recorder which is connected to the stimulation means.

Hitherto, proposed were various clinical cardiac electrophysiological methods which make use of heart stimulation inspection apparatus of the kind mentioned above. One of these methods is a so-called "over-drive method" in which a stimulating wave of a high frequency is continuously applied to the heart portion of the human body for a predetermined periods and the length of time from a moment at which the last electrical stimulation occurs till the moment at which the autochthonous beat is started, i.e. the so-called sine function recovery time, is measured. In another method known as "early stimulating method",a stimulation wave is applied such that the interval of the pulse is gradually decreased and the refractory period is measured. These methods make it possible to effectively inspect the function of sine knot and stimulation transmission function which cannot be derived from ordinary cardiograms.

Hitherto, it has been a common measure to keep the recorder operative throughout the measurement of the sine function recovery time or the refractory period. Alternatively, the user was obliged to manually start and stop the recorder in accordance with the timing of measurement while watching a clock after the start of the stimulation, in order to save recording paper. It was also necessary to hand-write the data concerning stimulation pattern on the intracardiac electrocardiogram, in order to record the stimulation pattern together with the intracardiac electrocardiogram. Consequently, the inspection required complicated and laborious works.

### SUMMARY OF THE INVENTION

Accordingly, an object of the present invention is to provide a stimulating heart inspection apparatus which is capable of effecting intercardiac pacing by a stimulation device and recording the intracardiac electrocardiogram on a recorder thereby enabling measurement and recording of the sine function recovery time or the refractory period, wherein the recording is conducted automatically so as to eliminate any necessity for the manual control of the recorder while saving recording paper.

According to the present invention, this object is solved by a heart stimulation inspection apparatus of the type specified above and comprising the following features: final stimulation signal generating means which are built-in in either the stimulation means or the recorder and capable of generating a final stimulation signal at a timing corresponding to the final electrical stimulation wave; recording start signal generating means which are built-in in the stimulation means and adapted for generating a recording start signal for starting the recorder in advance of the generation of the final electrical stimulation wave; recording stop signal generating means which are built-in in either the stimulation means or the recorder and capable of detecting the intracardiac electrocardiogram signal generated for the first time after the generation of the final electrical stimulation wave and generating a recording stop signal after the detection of the intracardiac electrocardiogram signal;
recording control means which are built-in in the recorder and capable of starting the recording paper feed in response to the recording start signal and stopping the recording paper feed in response to a recording stop signal; stimulation patter signal generating means which are built-in in the stimulation means and capable of generating a stimulation pattern signal representative of the stimulation pattern, which is converted into stimulation pattern data; and recording signal generating means for generating recording signals in accordance with which the waveforms of both the final stimulation signal and the intracardiac electrocardiogram signal, and the stimulation pattern data are recorded on the recording paper.

According to a further development of the apparatus according to the invention, the recording start signal generating means, the stimulation pattern signal generating means, the recording stop signal generating means, and the electrical stimulation wave generating means are constituted by a CPU circuit which is built-in in the stimulation means.

In a further development of the apparatus according to the invention, the recording signal generating means and the recording control means are constituted by a CPU circuit which is built-in in the recorder.

A still further development of the apparatus according to the present invention is characterized in that the recording start signal generating means are capable of generating the recording start signal at a moment which is several seconds ahead of the generation of the final electrical stimulation wave, and in that the recording stop signal generating means are capable of generating the recording stop signal at a moment which is several seconds after the rise of the amplitude of the first intracardiac electrocardiogram signal.

According to the invention, various labrious works for controlling the recorder are eliminated in the clinical cardiac electrophysiological inspection conducted for the purpose of measuring the sine function recovery time and the refractory period.

This contributes to a reduction in the man-hour as compared with the case where a conventional apparatus is used, while relieving the pain of a patient. In addition, the consumption of recording paper can be minimized.

### BRIEF DESCRIPTION OF THE DRAWINGS

Fig. 1 is a block diagram of an embodiment of the stimulating heart inspection apparatus in accordance with the present invention;
Fig. 2 is a block diagram of a stimulating heart inspection apparatus which is a more practical embodiment of the present invention; and
Fig. 3 is a diagram illustrating an example of a record obtained by using the embodiment shown in Fig. 2.

### DESCRIPTION OF THE PREFERRED EMBODIMENTS

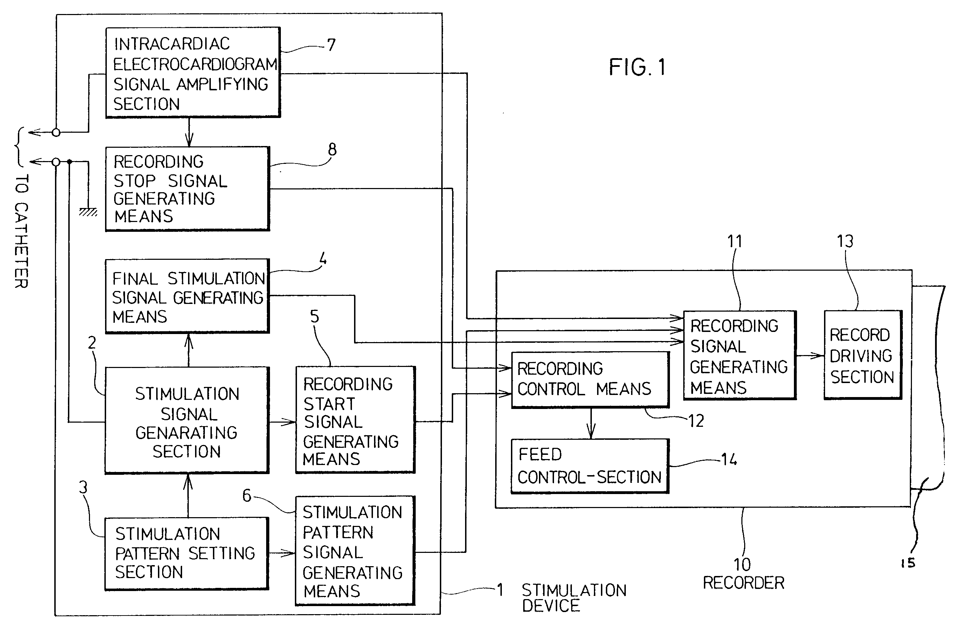

Referring to Fig. 1, an embodiment of the stimulating heart inspection apparatus of the present invention has a stimulation device 1 and a recorder 10. The stimulation device 1 has an electrical stimulation wave generating section 2 which generates, in accordance with a stimulation pattern externally set through a stimulation pattern setting section 3, an electrical stimulation wave in the form of a pulse train and applies the thus generated stimulation wave to the heart of a patient through a catheter in an insulating manner. The stimulation device 1 also has an intracardiac electrocardiogram signal amplifying section 7 which amplifies in an insulating manner the intracardiac electrocardiogram signal induced in the catheter. The stimulation device 1 further comprises the following components: a final stimulation signal generating means 4 which generates a signal at a timing corresponding to the final pulse of the electrical stimulation signal; a recording start signal generating means 5 for generating a signal for starting a recorder 10 in advance of the generation of the final stimulation signal; a stimulation pattern signal generating means 6 for generating a stimulation pattern signal representative of the stimulation pattern; and recording stop signal generating means 8 for generating a recorder stop signal upon detection of the first autochthonous beat.

The recorder 10 connected to the stimulation device 1 has a control unit 14 for controlling the feed of a recording paper 15 and a record driving unit 13 for recording characters and signal waveforms on the recording paper 15 in response to recording signals input thereto. The recorder 10 also incorporates a recording signal generating means 11 for recording the input final stimulation signal and intracardiac electrocardiogram waveforms, while causing the recorder 10 to print data corresponding to the stimulation pattern, and a recording control means 12 for starting the recording operation in response to a recording start signal and for stopping the recording operation in response to a recording stopping signal.

In the measurement of the sine function recovery time or the refractory period, the floating electrical stimulation signal generating section 2 in the stimulation device 1 applies electrical stimulation to the heart through the catheter in accordance with the stimulation pattern which has been externally set by the stimulation pattern setting section 3. The recording start signal generating means 5 delivers a recording start signal in response to a timing signal which is generated in advance of the delivery of the final electrical stimulation pulse. The final stimulation signal generating means 4 then delivers a final stimulation signal in synchronization with a timing signal which is generated when the final stimulation pulse is generated. The stimulation pattern signal generating means 6 then delivers a stimulation pattern signal corresponding to the externally set stimulation pattern, in response to the delivery of the recording start signal.

After the termination of the sine function recovery time or the refractory period, an autochthonous intracardiac electrocardiogram signal is induced in the catheter and is delivered through the intracardiac electrocardiogram signal amplifying section 7. Upon detection of the thus delivered intracardiac electrocardiogram signals the recording stop signal generating means 8 delivers the recording stop signal when a predetermined period is terminated after the detection of the intracardiac electrocardiogram signal. Meanwhile, the recording control means 12 in the recorder 10 operates in response to the recording start signal so as to activate at least the feed control unit 14 thereby to commence the feed of the recording paper 15. Then, the recording signal generating means 11 operates in response to the final stimulation signal so as to enable the record driving section 13 to record the waveform of the final stimulation signal on the recording paper 15. The recorder 10 continues to operate until the sine function recovery time of the refractory period expires as from the moment of generation of the final stimulation signal. When the first autochthonous beat is detected, the recording signal generating means 11 operates to record the waveform of the beat, and then the recording control means 12 operates in response to the recording stop signal thereby to terminate at least the paper feed.

The described embodiment can be modified such that the final stimulation signal generating means 4 is built-in in the recorder 10 so as to generate the final stimulation signal after termination of a predetermined period after the generation of the recording starting signal. The recording stop signal generating means 8 also may be built-in in the recorder 10 so as to generate the recording stop signal in response to an intracardiac electrocardiogram signal.

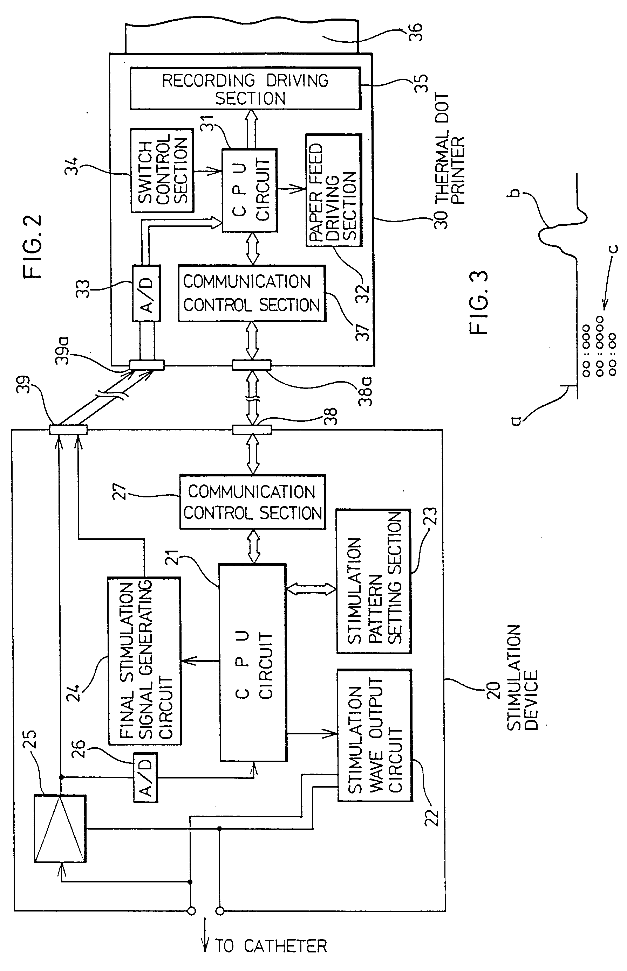

Fig. 2 illustrates a more practical embodiment of the stimulating heart inspection apparatus of the present invention. In this embodiment, a thermal dot printer 30 as the recorder 10 is connected to the stimulation device 20 through connectors 38, 38a and connectors 39, 39a.

The stimulation device 20 has a CPU (Central Processing Unit) circuit 21 having various circuits such as a ROM, a RAM, a clock generator, and so forth which constitute the recording start signal generating means 5, stimulation pattern signal generating means 6 and the recording stop signal generating means 8 which are mentioned in the description of the embodiment shown in Fig. 1. The CPU circuit 21 also cooperates with a stimulation wave output circuit 22 in constituting an electrical stimulation wave generating section 2. A reference numeral 23 denotes a keyboard-type stimulation pattern setting section through which the stimulation pattern having factors such as the kind of stimulation, stimulation interval, stimulation time and so forth are set. The stimulation pattern setting section 23 also delivers a code signal which represents the set key position. A reference numeral 24 designates a final stimulation signal generating circuit which is constituted by a pulse generating circuit adapted to be triggered by a timing signal from the CPU circuit 21, and is adapted for generating the final stimulation signal a. The stimulation device 20 further has an amplifier 25 for amplifying the intracardiac electrocardiogram signal induced in the catheter, an A/D converter 26 for converting the output of the amplifier 25 into digital signals, and a communication control section 27. The communication control section 27 is a device which meets the serial transmission standard of RS-232-C capable of conducting transmission of the digital signals such as a recording start command signal, recording stop command signal and stimulation pattern command signal, through a signal exchange between itself and the communication control section 37 of the thermal dot printer 30. The output portion of the stimulation wave output circuit 22 and the input portion of the amplifier 25 are electrostatically floating through an insulating coupling circuit such as a transformer.

The CPU circuit 21 is adapted for supplying the stimulation wave output circuit 22 with the stimulation wave signal of the pattern which has been set through the stimulation pattern setting section 23, thereby enabling the stimulation wave output circuit 22 to deliver to the catheter the stimulation wave which has been formed as a pulse train and which has been amplified. Then, a recording start command signal of a predetermined format is delivered through the communication control section 27 at a predetermined moment which is, for example, 3 seconds in advance of the generation of the final stimulation wave. Immediately after the delivery of the recording start signal, the CPU 21 delivers a stimulation pattern command signal of a predetermined format having factors such as the type of the stimulation pattern, stimulation interval, stimulation time and so forth. At the moment of generation of the final stimulation wave, the CPU circuit 21 delivers a trigger signal to the final stimulation signal generating circuit 24 thereby enabling the latter to produce the final stimulation signal a.

Subsequently, the CPU circuit 21 operates to pick-up the intracardiac electrocardiogram signal which is generated for the first time after the generation of the final stimulation wave, and detects the change in the amplitude of the intracardiac electrocardiogram signal b. The CPU circuit 21 then delivers a recording stop signal of a predetermined format to the communication control section 27, when a predetermined time, e.g., 5 minutes, has terminated after the rise of the intracardiac electrocardiogram signal b.

The thermal dot printer 30 also has a CPU circuit 31 which is adapted to receive signals such as a power on-off signal and a recording speed signal through a switch control section 34 and to receive also various command signals mentioned before through the communication control section 37. Thus, the CPU circuit 31 constitutes the recording signal generating means 11 and the recording control means 12 which were explained before in connection with Fig. 1. The printer 30 also has a paper feed driving section 32 which starts and stops the feed of the recording paper 36 in accordance with the signal from the CPU circuit 31, and a recording driving section 35 which drives the thermal head upon receipt of the dot recording signal from the CPU circuit 31.

The CPU circuit 31 receives a recording start command signal and a recording stop command signal from the communication control section 37, and delivers the paper feed start and stop signals to the paper fed driving section 32 to enable the start and stop of feed of the recording paper 36 upon reading these command signals.

The CPU circuit 31 is also operable for the purpose of converting the final stimulation signal a and the intracardiac electrocardiogram signal b which are supplied through an A/D converter 33 into dot recording signals corresponding to the waveforms of the respective signals.

The CPU circuit 31 also reads the stimulation pattern command signal and reads the dot recording signals corresponding to one-line character data corresponding to the stimulation pattern from a memory. The thus read dot recording signal is supplied to the recording driving section 35.

This stimulating heart inspection apparatus operates in a manner which will be explained hereinafter.

A desired stimulation pattern is selected through the stimulation pattern setting section 23 in the stimulation device 20. Then, the CPU circuit 21 forms a stimulation wave signal on the basis of the clocks and delivers this signal to the stimulation wave output circuit 22 to enable the circuit 22 to apply an electrical stimulation wave, through the catheter, for example to the atrium of a heart. Meanwhile, the power supply to the thermal dot printer 30 has been turned on by the operation of the switch control section 34, and the recording speed signal also has been set beforehand. Then, the recording start command signal is supplied to the CPU circuit 31 of the thermal dot printer 30 through the communication control section 27 at a moment which is 3 seconds ahead of the final stimulation wave. Upon reading this command signals the CPU circuit 31 operates to start the feed of the recording paper right away. After elapse of further 3 seconds, the final stimulation signal a is supplied and changed into a digital signal through the A/D converter 33. The digital signal is then changed by the CPU circuit 31 into dot recording signals in accordance with which dot printing elements of the recording head are driven by the recording driving section 35, whereby the waveform is recorded as illustrated in Fig. 3. For instances the first autochthonous intracardiac electrocardiogram signal b is received after elapse of 2 seconds from the start of the recordings and the thus received signal b is converted by the CPU circuit 31 into dot wave signal, whereby the waveform of this signal is written on the recording paper 36. Upon detection of the intracardiac electrocardiogram signal b, the CPU circuit 21 generates a recording stop command signal at a moment which is 5 seconds after the detection of the signal b. In response to this stop command signal, the CPU circuit 31 of the printer 30 operates to stop the feed of the recording paper 36. Before stopping the feed of the recording paper 36, the CPU circuit 31 operates to convert the stimulation command signal into dot recording signals so as to enable the stimulation pattern data c to be printed. Although the invention has been described through specific forms, it is to be understood that the described embodiments are only illustrative and various changes and modifications may be imparted thereto without departing from the scope of the invention.

It is also possible to arrange such that the recording start signal generating means 5 does not only control the paper feed but also handles other types of control such as the control of the recording control means 12 by a command signal which appoints the recording pattern corresponding to the stimulation pattern.

## Claims

1. A heart stimulation inspection apparatus including stimulation means (1, 20) in which electrical stimulating waves, generated by electrical stimulation wave generating means (2), for effecting pacing in accordance with an externally set stimulation pattern, are input through a catheter and the intracardiac electrocardiogram signals induced in the catheter are amplified and recorded on a recorder (10, 30) which is connected to the stimulation means (1, 20),
characterized by the following features:
- final stimulation signal generating means (4) which are built-in in either the stimulation means (1, 20) or the recorder (10, 30) and capable of generating a final stimulation signal (a) at a timing corresponding to the final electrical stimulation wave,
- recording start signal generating means (5) which are built-in in the stimulation means (1, 20) and adapted for generating a recording start signal for starting the recorder (10, 30) in advance of the generation of the final electrical stimulation wave,
- recording stop signal generating means (8) which are built-in in either the stimulation means (1, 20) or the recorder (10, 30) and capable of detecting the intracardiac electrocardiogram signal (b) generated for the first time after the generation of the final electrical stimulation wave and generating a recording stop signal after the detection of the intracardiac electrocardiogram signal (b),
- recording control means (12) which are built-in in the recorder (10, 30) and capable of starting the recording paper feed in response to the recording start signal and stopping the recording paper feed in response to a recording stop signal,
- stimulation pattern signal generating means (6) which are built-in in the stimulation means (1, 20) and capable of generating a stimulation pattern signal representative of the stimulation pattern, which is converted into stimulation pattern data (c), and
- recording signal generating means (11) for generating recording signals in accordance with which the waveforms of both the final stimulation signal (a) and the intracardiac electrocardiogram signal (b), and the stimulation pattern data (c) are recorded on the recording paper.

2. The apparatus according to claim 1,
characterized in that the recording start signal generating means (5), the stimulation pattern signal generating means (6), the recording stop signal generating means (8), and the electrical stimulation wave generating means (2) are constituted by a CPU circuit (21) which is built-in in the stimulation means (20).

3. The apparatus according to claim 1 or 2,
characterized in that the recording signal generating means (11) and the recording control means (12) are constituted by a CPU circuit (31) which is built-in in the recorder (30).

4. The apparatus according to any of claims 1 to 3,
characterized in that the recording start signal generating means (5) are capable of generating the recording start signal at a moment which is several seconds ahead of the generation of the final electrical stimulation wave, and in that the recording stop signal generating means (8) are capable of generating the recording stop signal at a moment which is several seconds after the rise of the amplitude of the first intracardiac electrocardiogram signal (b).

## Patentansprüche

1. Herzstimulations-Prüfvorrichtung, die eine Stimulationseinrichtung (1, 20) aufweist, wobei elektrische Stimulationswellen, die von einer Erzeugungseinrichtung (2) für elektrische Stimulationswellen erzeugt werden, um eine Anregung nach Maßgabe eines extern eingestellten Stimulationsmusters zu bewirken, durch einen Katheter angelegt werden und die intrakardialen EKG-Signale, die in dem Katheter induziert werden, verstärkt und mit einem Aufzeichnungsgerät (10, 30), das mit der Stimulationseinrichtung (1, 20) verbunden ist, aufgezeichnet werden, gekennzeichnet durch die folgenden Merkmale:
- eine Erzeugungseinrichtung (4) für endgültige Stimulationssignale, die entweder in die Stimulationseinrichtung (1, 20) oder das Aufzeichnungsgerät (10, 30) eingebaut und fähig ist, ein endgültiges Stimulationssignal (a) mit einer Zeitsteuerung zu erzeugen, die der endgültigen elektrischen Stimulationswelle entspricht,
- eine Aufzeichnungsstartsignal-Erzeugungseinrichtung (5), die in die Stimulationseinrichtung (1, 20) eingebaut und dafür ausgelegt ist, ein Aufzeichnungsstartsignal zum Starten des Schreibers (10, 30) vor der Erzeugung der endgültigen elektrischen Stimulationswelle zu erzeugen,
- eine Aufzeichnungsstoppsignal-Erzeugungseinrichtung (8), die entweder in die Stimulationseinrichtung (1, 20) oder das Aufzeichnungsgerät (10, 30) eingebaut und fähig ist, das intrakardiale EKG-Signal (b) zu detektieren, das zum ersten Mal nach der Erzeugung der endgültigen elektrischen Stimulationswelle erzeugt wird, und nach dem Detektieren des intrakardialen EKG-Signals (b) ein Aufzeichnungsstoppsignal zu erzeugen,
- eine Aufzeichnungssteuereinrichtung (12), die in das Aufzeichnungsgerät (10, 30) eingebaut und fähig ist, den Aufzeichnungspapiervorschub aufgrund des Aufzeichnungsstartsignals zu starten und den Aufzeichnungspapiervorschub aufgrund eines Aufzeichnungsstoppsignals zu stoppen,
- eine Stimulationsmustersignal-Erzeugungseinrichtung (6), die in die Stimulationseinrichtung (1, 20) eingebaut und fähig ist, ein Stimulationsmustersignal zu erzeugen, das für das Stimulationsmuster repräsentativ ist, das in Stimulationsmusterdaten (c) umgewandelt wird, und
- eine Aufzeichnungssignal-Erzeugungseinrichtung (11), um Aufzeichnungssignale zu erzeugen, gemäß denen die Wellenformen sowohl des endgültigen Stimulationssignals (a) als auch des intrakardialen EKG-Signals (b) und die Stimulationsmusterdaten (c) auf dem Aufzeichnungspapier aufgezeichnet werden.

2. Vorrichtung nach Anspruch 1,
dadurch gekennzeichnet, daß die Aufzeichnungsstartsignal-Erzeugungseinrichtung (5), die Stimulationsmustersignal-Erzeugungseinrichtung (6), die Aufzeichnungsstoppsignal-Erzeugungseinrichtung (8) und die Erzeugungseinrichtung (2) für elektrische Stimulationswellen von einer CPU-Schaltung (21) gebildet sind, die in die Stimulationseinrichtung (20) eingebaut ist.

3. Vorrichtung nach Anspruch 1 oder 2,
dadurch gekennzeichnet, daß die Aufzeichnungssignal-Erzeugungseinrichtung (11) und die Aufzeichnungssteuereinrichtung (12) von einer CPU-Schaltung (31) gebildet sind, die in das Aufzeichnungsgerät (30) eingebaut ist.

4. Vorrichtung nach einem der Ansprüche 1 bis 3,
dadurch gekennzeichnet, daß die Aufzeichnungsstartsignal-Erzeugungseinrichtung (5) fähig ist, das Aufzeichnungsstartsignal in einem Moment zu erzeugen, der mehrere Sekunden vor der Erzeugung der endgültigen elektrischen Stimulationswelle liegt,
und daß die Aufzeichnungsstoppsignal-Erzeugungseinrichtung (8) fähig ist, das Aufzeichnungsstoppsignal in einem Moment zu erzeugen, der mehrere Sekunden nach dem Anstieg der Amplitude des ersten intrakardialen EKG-Signals (b) liegt.

## Revendications

1. Appareil d'inspection de stimulation cardiaque comprenant des moyens de stimulation (1, 20) dans lesquels des ondes stimulantes électriques, produites par des moyens de production d'ondes de stimulation électriques (2) afin d'effectuer un cadencement en accord avec un modèle de stimulation dicté depuis l'extérieur, sont introduites à travers un cathéter et les signaux d'électrocardiogramme intracardiaques produits dans le cathéter sont amplifiés et enregistrés dans un appareil d'enregistrement (10, 30) qui est connecté aux moyens de stimulation (1, 20),
caractérisé par les éléments suivants :
- des moyens produisant le signal final de stimulation (4), qui sont intégrés soit dans les moyens de stimulation (1, 20) soit dans l'appareil d'enregistrement (10, 30) et capables de produire un signal final de stimulation (a) à une temporisation correspondante à l'onde finale de stimulation électrique,
- des moyens produisant le signal de démarrage d'enregistrement (5), qui sont intégrés dans les moyens de stimulation (1, 20) et adaptés à produire un signal de démarrage d'enregistrement pour démarrer l'appareil d'enregistrement (10, 30) en avance par rapport à la production de l'onde finale de stimulation électrique,
- des moyens produisant un signal d'arrêt d'enregistrement (8), qui sont intégrés soit dans les moyens de stimulation (1, 20) soit dans l'appareil d'enregistrement (10, 30) et capables de détecter le signal électrocardiogramme intracardiaque (b) produit pour la première fois après la production de l'onde finale de stimulation électrique et produisant un signal d'arrêt d'enregistrement après la détection du signal d'électrocardiogramme intracardiaque (b),
- des moyens de commande d'enregistrement (12) qui sont intégrés dans l'appareil d'enregistrement (10, 30) et capables de démarrer l'alimentation de papier d'enregistrement en réponse au signal de démarrage d'enregistrement et d'arrêter l'alimentation de papier d'enregistrement en réponse au signal d'arrêt d'enregistrement,
- des moyens (6) produisant un signal correspondant à un modèle de stimulation, qui sont intégrés dans les moyens de stimulation (1, 20) et capables de produire un signal représentatif du modèle de stimulation, qui est converti en données (c) correspondant au modèle de stimulation, et
- des moyens produisant un signal d'enregistrement (11) pour produire des signaux d'enregistrement en accord desquels les formes d'onde du signal final de stimulation (a) et ainsi que du signal électrocardiogramme intracardiaque (b), et les données (c) correspondant au modèle de stimulation sont enregistrés sur le papier d'enregistrement.

2. Appareil selon la revendication 1,
caractérisé en ce que les moyens produisant le signal de démarrage d'enregistrement (5), les moyens produisant le signal correspondant à un modèle de stimulation (6), les moyens produisant le signal d'arrêt d'enregistrement (8) et les moyens produisant l'onde de stimulation électrique (2) sont constitués par un circuit d'unité centrale (CPU) (21) qui est intégré dans les moyens de stimulation (20).

3. Appareil selon l'une ou l'autre des revendications 1 et 2, caractérisé en ce que les moyens produisant le signal d'enregistrement (11) et les moyens de commande d'enregistrement (12) sont constitués par un circuit d'unité centrale (CPU) (31) qui est intégré dans l'appareil d'enregistrement (30).

4. Appareil selon l'une quelconque des revendications 1 à 3, caractérisé en ce que les moyens produisant le signal de démarrage d'enregistrement (5) sont capables de produire le signal de démarrage d'enregistrement à un moment se trouvant quelques secondes avant la production de l'onde finale de stimulation électrique, et en ce que les moyens produisant le signal d'arrêt d'enregistrement (8), sont capables de produire le signal d'arrêt d'enregistrement à un moment se trouvant quelques secondes après la montée de l'amplitude du premier signal électrocardiogramme intracardiaque.
